(19) Europäisches Patentamt
European Patent Office
Office européen des brevets

(11) **EP 2 668 895 A1**

(12) **EUROPEAN PATENT APPLICATION**

(43) Date of publication:
**04.12.2013 Bulletin 2013/49**

(51) Int Cl.:
***A61B 5/00*** *(2006.01)*     ***G01B 9/02*** *(2006.01)*

(21) Application number: **13170094.0**

(22) Date of filing: **31.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **01.06.2012 KR 20120059429**

(71) Applicant: **Samsung Electronics Co., Ltd Gyeonggi-do 443-742 (KR)**

(72) Inventors:
- **Lim, Jae-guyn Gyeonggi-do (KR)**
- **Lee, Seong-deok Gyeonggi-do (KR)**
- **Jang, Woo-young Gyeonggi-do (KR)**

(74) Representative: **Grünecker, Kinkeldey, Stockmair & Schwanhäusser Leopoldstrasse 4 80802 München (DE)**

(54) **Apparatus and method for generating tomographic image**

(57)     A method of generating a tomographic includes detecting a coherence signal that is phase-modulated in a first direction with respect to a cross-section of a subject and includes cross-sectional information of the subject as raw data about the subject; generating a reference temporary tomographic image and at least one temporary tomographic image by performing signal processing on the raw data; detecting an artifact are of the reference temporary tomographic image based on a result of comparing the reference temporary tomographic image with the at least one temporary tomographic image and based on artifact statistics regarding whether an artifact exists; and restoring the artifact area.

FIG. 2

```
DETECT COHERENCE SIGNAL INCLUDING CROSS-
SECTION INFORMATION OF SUBJECT THAT IS
PHASE-MODULATED IN FIRST DIRECTION WITH      — S220
RESPECT TO CROSS-SECTION OF SUBJECT, AS
RAW DATA ABOT SUBJECT

PERFORM SIGNAL PROCESSING ON RAW DATA
TO GENERATE REFERENCE TEMPORARY           — S240
TOMOGRAPHIC IMAGE AND AT LEAST ONE
TEMPORARY TOMOGRAPHIC IMAGE

DETECT ARTIFACT AREA OF REFERENCE
TEMPORARY TOMOGRAPHIC IMAGE BASED ON
RESULT OF COMPARING REFERENCE TEMPORARY   — S260
TOMOGRAPHIC IMAGE AND AT LEAST ONE
TEMPORARY TOMOGRAPHIC IMAGE
AND STATISTICS WITH RESPECT TO ARTIFACT AREA

RESTORE ARTIFACT AREA                       — S280
```

EP 2 668 895 A1

**Description**

BACKGROUND

1. Field

This application relates to apparatuses and methods for generating high-quality tomographic images by detecting and restoring an artifact area.

2. Description of Related Art

Tomography is a technique for obtaining tomographic images of a subject through the use of a penetrating wave. Tomography is used in various fields, and demands for high-quality tomographic images have increased. In particular, in human medical fields, the technology of generating tomographic images accurately based on limited resources is an important issue.

SUMMARY

In one general aspect, a method of generating a tomographic image includes detecting a coherence signal that is phase-modulated in a first direction with respect to a cross-section of a subject and may include cross-sectional information of the subject as raw data about the subject; generating a reference temporary tomographic image and at least one temporary tomographic image by performing signal processing on the raw data; detecting an artifact area of the reference temporary tomographic image based on a result of comparing the reference temporary tomographic image with the at least one temporary tomographic image and based on artifact statistics regarding whether an artifact exists; and restoring the artifact area in response to a result of the detecting. Accordingly a high-quality tomographic image may be generated accurately without increasing a complexity of a calculation required for generation of the tomographic image.

The generating of a reference temporary tomographic image and at least one temporary tomographic image by performing signal processing on the raw data may include generating demodulated data by demodulating the raw data; and performing signal processing on the demodulated data to convert the demodulated data into the reference temporary tomographic image and the at least one temporary tomographic image. Thus, the reference temporary tomographic image and the at least one temporary tomographic image may be obtained by converting demodulated data obtained by demodulating the raw data.

The generating of demodulated data may include adjusting at least one parameter of a filter function defining a vestigial sideband (VSB) filter. The VSB filter function represents single sideband filtering wherein the VSB is only party cut-off or suppressed which is particularly useful for phase-shift keying.

The at least one parameter may be a duration or a roll-off value of the filter function. These represent suitable parameters for adjusting the filter function. The duration represents the width while the roll-off represents the steepness of the filter function.

The generating of demodulated data may include generating the raw data into at least two pieces of demodulated data.

The performing of signal processing on the demodulated data may include performing signal processing on the at least two pieces of demodulated data to convert the at least two pieces of demodulated data into the reference temporary tomographic image and the at least one temporary tomographic image. Hence, the reference temporary tomographic image and the at least one temporary tomographic image may be obtained from separate respective pieces of demodulated data.

The generating of demodulated data may include demodulating the at least two pieces of demodulated data using different VSB filters defined by different parameters. Thus, optimized parameters may be used for the respective VSB filters.

The performing of signal processing on the demodulated data may include performing signal processing on the demodulated data to convert the one demodulated data into the reference temporary tomographic image and the at least one temporary tomographic image. In this case, only one demodulated data set is necessary.

The performing of the signal processing on the demodulated data may include performing signal processing on the demodulated data in a second direction that is perpendicular to the first direction. Thereby it is possible to generate a tomographic image showing structures also in a second direction.

The demodulated data may be in a wavelength domain; and the performing of signal processing on the demodulated data may include converting the demodulated data in the wavelength domain into depth information about the subject. Accordingly, depth information is obtained with respect to the subject.

The detecting of an artifact area of the reference temporary tomographic image may include defining the artifact area based on a difference between gradient information of the reference temporary tomographic image and gradient information of the at least one temporary tomographic image. This is a very sensitive method for defining the artifact area.

The detecting of an artifact area of the reference temporary tomographic image may include defining the artifact area based on a difference between image intensities of the reference temporary tomographic image and image intensities of the at least one temporary tomographic image. This is a very efficient and fast method for defining the artifact area.

The restoring of the artifact area may include restoring the artifact area based on a first weight that is applied to a distance between the artifact area and at least one adjacent pixel and a second weight that is applied to a result of comparing the reference temporary tomographic image with the at least one temporary tomographic image with respect to the artifact area. This is particularly useful way for restoring of the artifact area.

The method may further include generating the reference temporary tomographic image with the restored artifact as a final tomographic image with respect to the cross-section of the subject. Hence, a final tomographic image that can be stored is obtained.

The method may be an optical coherent tomographic (OCT) method. This corresponds to a particularly advantageous signal acquisition and processing method, in particular with respect to signal-to-noise ratio.

In another general aspect, a non-transitory computer readable storage medium stores a program for controlling a computer to perform the method of generating a tomographic image discussed above.

In another general aspect, an apparatus for generating a tomographic image includes an image processing unit configured to receive raw data corresponding to a coherence signal including sectional information of the subject that is phase-modulated in a first direction with respect to a cross-section of the subject, and generate a reference temporary tomographic image and at least one temporary tomographic image by performing signal processing on the raw data; and an artifact processing unit configured to detect an artifact area of the reference temporary tomographic based on a result of comparing the reference temporary tomographic image with the at least one temporary tomographic image and based on artifact statistics regarding whether an artifact exists, and restore the artifact area. The advantages of the apparatus for generating a tomographic image according to the invention as well as the following further developments correspond to the advantages mentioned above with respect to the method according to the invention.

The artifact processing unit may include an artifact area determining unit configured to define the artifact area based on a difference between gradient information of the reference temporary tomographic image and gradient information of the at least one temporary tomographic image; and an artifact area restoring unit configured to restore the artifact area based on a first weight that is applied to a distance between the artifact area and at least one adjacent pixel and a second weight that is applied to a result of comparing the reference temporary tomographic image with the at least one temporary tomographic image with respect to the artifact area.

The artifact area determining unit may include at least one vestigial sideband (VSB) filter.

The artifact area restoring unit may be further configured to generate the reference temporary tomographic with the restored artifact area as a final tomographic image with respect to the cross-section of the subject.

Other features and aspects will be apparent from the following detailed description, the drawings, and the claims.

BRIEF DESCRIPTION OF THE DRAWINGS

FIG. 1 is a block diagram illustrating an example of a tomographic image generating apparatus.

FIG. 2 is a flowchart illustrating an example of a method of generating a tomographic image.

FIG. 3 illustrates an example of generating a coherence signal of FIG. 1 or FIG. 2.

FIG. 4 is a detailed view of an example of a coherence system illustrated in FIG. 3.

FIG. 5 illustrates an example of an operation of a phase modulator illustrated in FIG. 3.

FIG. 6 illustrates an example of an image processing unit of FIG. 1.

FIG. 7 illustrates another example of an image processing unit of FIG. 1.

FIGS. 8A and 8B illustrate examples of demodulators illustrated in FIGS. 6 and 7.

FIGS. 9A and 9B are images showing an example of a relationship between an increase in a complexity of filtering and a folding phenomenon.

FIG. 10 is a flowchart illustrating an example of an operation of generating a reference temporary tomographic image and at least one temporary tomographic image illustrated in FIG. 2.

FIG. 11 is a flowchart illustrating an example of an operation of detecting an artifact area illustrated in FIG. 2.

FIG. 12 is a flowchart illustrating an example of an operation of detecting an artifact area illustrated in FIG. 2.

FIG. 13 illustrates an example of an operation of an artifact processing unit of FIG. 1 performing the method of FIG. 11 or FIG. 12.

FIG. 14 is a graph showing an example of a standard used by an artifact area determining unit of FIG. 1 to determine an artifact area.

FIG. 15 is a flowchart illustrating an example of an operation of restoring an artifact area illustrated in FIG. 2.

FIG. 16 illustrates examples of graphs showing a first weight and a second weight of FIG. 15.

FIG. 17 is a graph illustrating an example of adjusting a parameter of a filter function defining a VSB filter used in demodulating data.

FIGS. 18A-18C illustrate an example of an operation of restoring an artifact.

DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent to one of ordinary skill in the art. The sequences of operations described herein are merely examples, and are not limited to those set forth herein, but may be changed as will be apparent to one of ordinary skill in the art, with the exception of operations necessarily occurring in a certain order. Also, description of functions and constructions that are well known to one of ordinary skill in the art may be omitted for increased clarity and conciseness.

Throughout the drawings and the detailed description, the same reference numerals refer to the same elements. The drawings may not be to scale, and the relative size, proportions, and depiction of elements in the drawings may be exaggerated for clarity, illustration, and convenience.

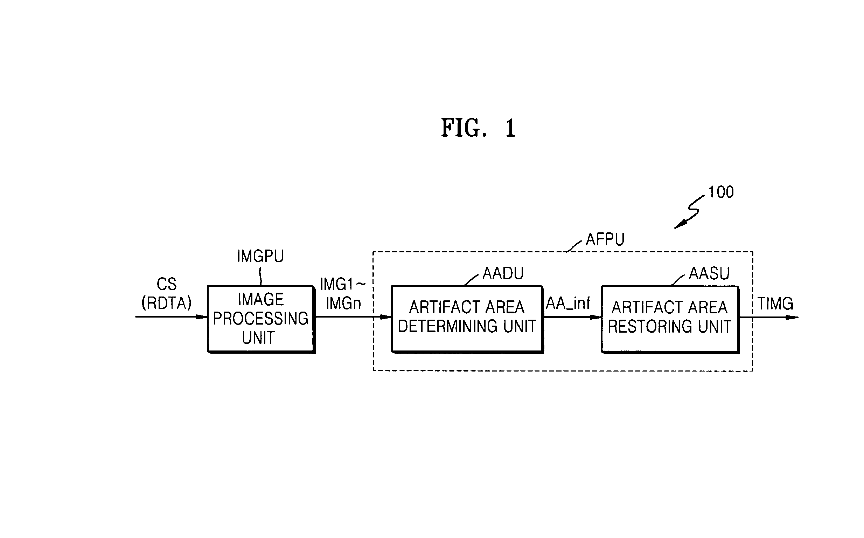

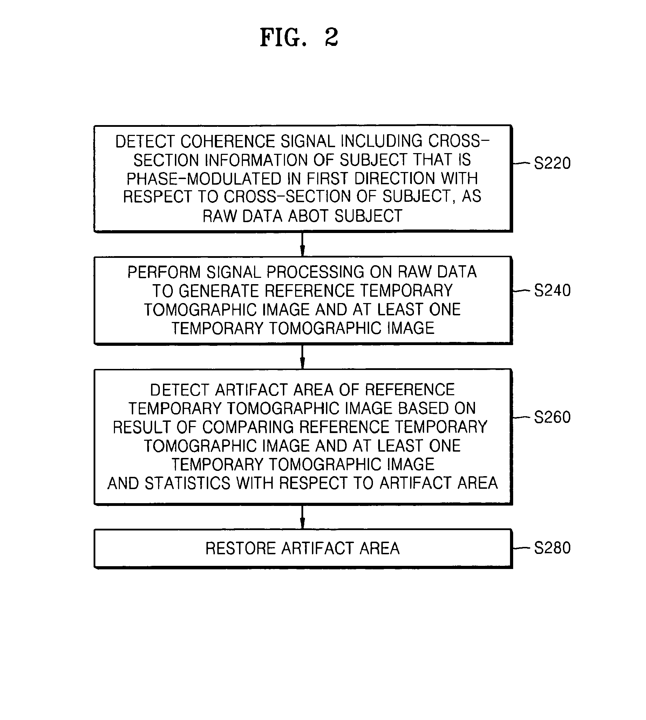

FIG. 1 is a block diagram illustrating an example of a tomographic image generating apparatus 100, and FIG. 2 is a flowchart illustrating an example of a method of generating a tomographic image.

Referring to FIGS. 1 and 2, the tomographic image generating apparatus 100 includes an image processing unit IMGPU and an artifact processing unit AFPU. The image processing unit IMGPU receives raw data RDTA that is detected according to a coherence signal CS in operation S220, and performs image processing on the raw data RDTA to generate at least two temporary tomographic images IMG1 through IMGn (n is a natural number of 2 or greater) in operation S240. For example, one of the at least two tomographic images IMG1 through IMGn may be a reference temporary tomographic image. For example, a first temporary tomographic image IMG1 may be the reference temporary tomographic image.

The artifact processing unit AFPU includes an artifact area determining unit AADU and an artifact area restoring unit AASU. The artifact area determining unit AADU compares the at least two temporary tomographic images IMG1 through IMGn and defines an artifact area in operation S260. As described above, one of the at least two temporary tomographic images IMG1 through IMGn may include a reference temporary tomographic image. In this case, the artifact area determining unit AADU compares a reference temporary tomographic image with the other temporary tomographic image or images of the at least two temporary tomographic images IMG1 through IMGn. In addition, an artifact area of the reference temporary tomographic image is detected based on a result of comparing the reference temporary tomographic image and at least one temporary tomographic image and artifact statistics regarding the presence of an artifact.

The artifact area restoring unit AASU restores the artifact area of the reference temporary tomographic image based on information about an artifact area (hereinafter, "artifact area information AA_Inf") that is defined by and received from the artifact area determining unit AADU in operation S280.

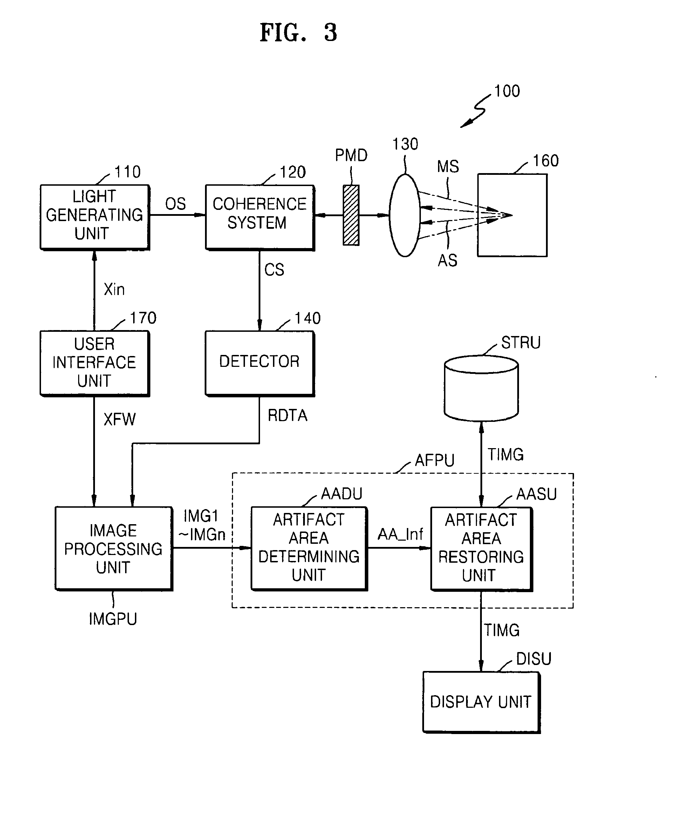

FIG. 3 illustrates an example of generating a coherence signal of FIG. 1 or FIG. 2.

Referring to FIGS. 1 and 3, the tomographic image generating apparatus 100 includes a light generating unit 110, a coherence system 120, a phase modulator PMD, and a detector 140 to generate a coherence signal CS. The tomographic image generating apparatus 100 of FIG. 3 may be an optical coherent tomographic (OCT) apparatus. However, the tomographic image generating apparatus 100 is not limited thereto. The tomographic image generating apparatus 100 may also be another type of medical imaging device. For example, the tomographic image generating apparatus 100 may be not only an OCT apparatus but also a medical imaging device including a vestigial sideband (VSB) filter, an example of which will be described later.

The light generating unit 110 generates an optical signal (OS). For example, the light generating unit 110 may emit an optical signal OS in response to an interface signal Xin corresponding to an input to a user interface unit 170. The user interface 170 may typically be an input device such as a keyboard, a mouse, or the like. Alternatively, the user interface 170 may be a graphical user interface (GUI) displayed on a display unit DISU. An event generated on the user interface 170 may be generated as an interface signal Xin. An event generated on the user interface 170 may be a keyup or a keydown if the user interface 170 is a keyboard. If the user interface 170 is a mouse, the event may be a click, and if the user interface 170 is a GUI, the event may be a touch.

Examples of the optical signal OS generated from the light generating unit 110 include a superluminescent diode (SLD) signal and an edge-emitting light emitting diode (ELED) signal. However, the optical signal OS is not limited thereto, and other types of optical signals may be used as an optical signal. The optical signal OS generated by the light generating unit 110 is transmitted to the coherence system 120. The optical signal OS may be transmitted to the coherence system 120 via free space, or may be transmitted to the coherence system 120 via a transmission medium. An example of the transmission medium is an optical fiber.

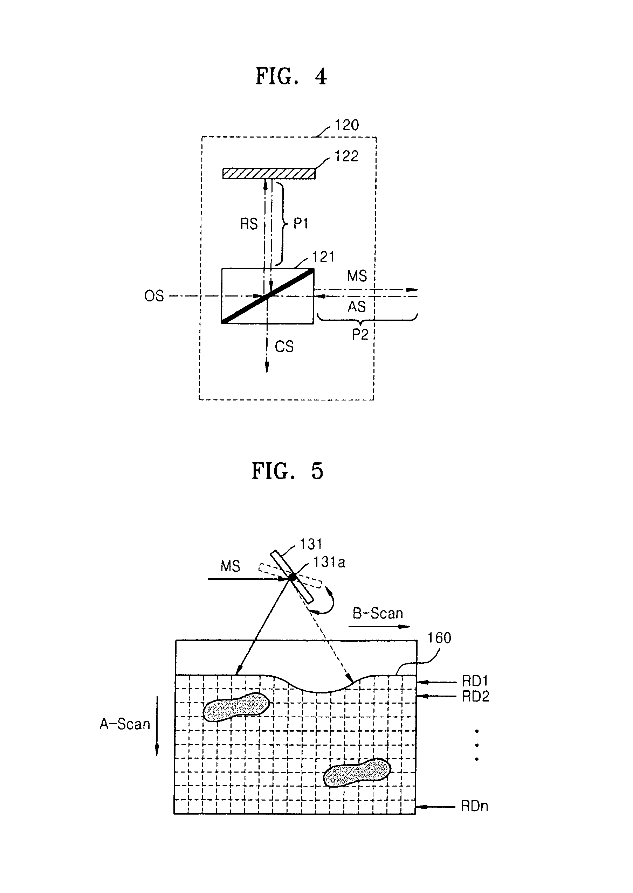

FIG. 4 is a detailed view of an example of the coherence system 120 illustrated in FIG. 3.

Referring to FIGS. 3 and 4, the coherence system 120 receives an optical signal OS and separates the optical signal OS into a measurement signal MS and a reference signal RS. To this end, in the coherence system 120, at least two different signal paths P1. and P2 are formed. The measurement signal MS separated from the optical signal OS is transmitted through one of the signal paths P1 and P2, for example, the signal path P2, and the reference signal RS is transmitted through the other signal path, for example, the signal path P1.

The coherence system 120 may separate an optical signal OS into a measurement signal MS and a reference signal RS at a splitting ratio. The splitting ratio may be defined by a rate of an output intensity of the measurement signal MS to an output intensity of the reference signal RS. For example, the coherence system 120 may separate an optical signal OS into a measurement signal MS and a reference signal RS at a splitting ratio of 5.5, or a splitting ratio of 9:1, or any of other splitting ratios. When separating a measurement signal MS and a reference signal RS using a beam splitter 121 as illustrated in FIG. 4, the splitting ratio may be determined according to transmission and reflection characteristics of the beam splitter 121.

Referring again to FIG. 1, the coherence system 120 transmits the measurement signal MS to the phase modulator PMD, and a probe 130 irradiates the measurement signal MS modulated by the phase modulator PMD onto a subject 160. The measurement signal MS irradiated from the probe 130 is reflected or scattered inside the subject 160.

FIG. 5 illustrates an example of an operation of the phase modulator PMD illustrated in FIG. 3. In FIG. 5, for convenience of description, the probe 130 irradiating the phase-modulated measurement signal MS onto the subject 160 is omitted.

Referring to FIGS. 3 and 5, the phase modulator PMD includes a scanning mirror 131. The scanning mirror 131 rotates about an axis 131a that is offset with respect to the measurement signal MS. As the scanning mirror 131 rotates about the axis 131 a, the measurement signal MS is scanned in a first direction B-scan of the subject 160 a lateral direction or a row direction) and is phase-modulated due to the offset between the axis 131a and the measurement signal MS. For example, the scanning mirror 131 rotates in units of one pixel in the row direction of the subject 160, thereby moving the measurement signal MS in the row direction of the subject 160 at a rate of one pixel to be irradiated. As a result, the measurement signal MS is phase-modulated in units of one pixel. The phase modulator PMD in this example may be a galvano scanner, and in this case, the B-scan direction refers to a direction due to a rotation of the galvano scanner.

The measurement signal MS that is irradiated onto the subject 160 in units of one pixel in the row direction is reflected or scattered in a second direction A-scan (a vertical direction or a column direction). In the example in FIG. 5, the second direction A-scan is perpendicular to the first direction B-scan of the subject 160, but is not limited to the perpendicular direction.

The measurement signal MS that is reflected or scattered is transmitted to the coherence system 120 as a response signal AS. For example, the response signal AS may be transmitted to the coherence system 120 along the same path as a path through which the measurement signal MS is irradiated onto the subject 160. Alternatively, the response signal AS may be transmitted to the coherence system 120 through a different path from the path along which the measurement signal MS is irradiated to the subject 160. Like transmission of the measurement signal MS, the response signal AS may be transmitted to the coherence system 120 through free space, or may be transmitted to the coherence system 120 via a transmission medium such as an optical fiber.

The coherence system 120 generates a coherence signal CS from interference between the response signal AS and the reference signal RS. In greater detail, the reference signal RS is transmitted through the signal path P1 inside the coherence system 120, and is reflected by a standard mirror 122 to be transmitted to the beam splitter 121. A portion of the reference signal RS transmitted to the beam splitter 121 is reflected by the beam splitter 121, and the other portion of the reference signal RS passes through the beam splitter 121. The reference signal RS that has passed through the beam splitter 121 interferes with the response signal AS that is reflected by the beam splitter 121 to generate the coherence signal CS.

Referring again to FIG. 1, coherence signal CS is input to the detector 140. The detector 140 detects the coherence signal CS generated by interference between the response signal AS and the reference signal RS as raw data RDTA in units of frames. Raw data that is formed in unit of frames may be obtained simultaneously by one rotation of the scan mirror 131, or first through m-th columns CD1-CDm of the raw data RDTA may be obtained sequentially by repeated rotation of the scan mirror 131 a number of times corresponding to the number of pixels in the first direction B-scan.

Referring again to FIG. 3, for example, the detector 140 detects a light intensity I of the coherence signal CS with respect to the subject 160 in units of rows as expressed by Equation 1 below. In Equation 1, $I_r$ denotes a light intensity of the reference signal RS, $I_s$ denotes a light intensity of the response signal AS, k denotes a wavelength, and $Z_{rs}$ denotes a difference between path lengths of the reference signal RS and the response signal AS (or depth information of the subject 160). Also, x denotes the number of pixels in the first direction B-scan (the row direction), and $f_B{}^*x$ denotes a phase-modulated value.

$$I(k, x) = \sum_{i=0}^{N} 2 \cdot \sqrt{I_r I_{s_i}} \cdot \cos\left(2k \cdot z_{rs_i} + f_B \cdot x\right) \qquad (1)$$

The detector 140 may detect a light intensity I of the coherence signal CS using a light receiving unit (not shown), and examples of the light receiving unit may include a photodetector. While a method of detecting a light coherence signal has been described above, a method of generating a tomographic image is not limited to generating a tomographic image from a light coherence signal, and other signals indicating information about tomographic images of a subject and the signals may be analyzed to generate tomographic images of the subject.

Raw data RDTA detected using the detector 140 is transmitted to the image processing unit IMGPU to generate at least two tomographic images.

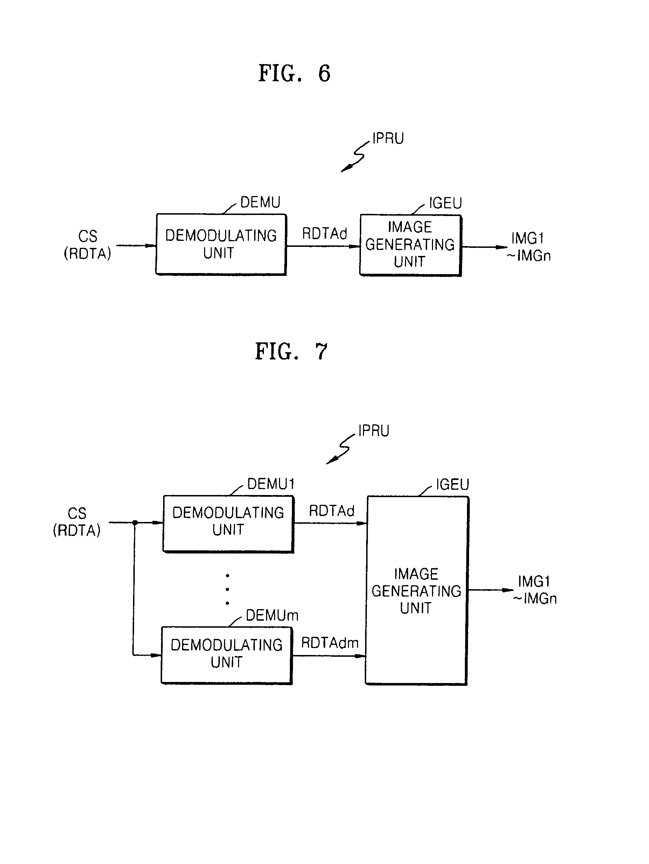

FIG. 6 illustrates an example of an image processing unit IMGPU of FIG. 1.

Referring to FIGS. 1 and 6, the image processing unit IMGPU in this example may include a demodulator DEMU and an image generating unit IGEU. The demodulator DEMU may demodulate raw data RDTA in units of 1 piece of data to units of 1 piece of demodulated data RDTAd. For example, the demodulator DEMU of FIG. 6 described later may demodulate one piece of raw data RDTA to one piece of demodulated data RDTAd. The image generating unit IGEU may perform signal processing on the raw data RDTA. For example, when demodulating of the raw data RDTA is performed in units of rows in the first direction B-scan, signal processing in the image generating unit IGEU may be performed in units of columns in the second direction A-scan. The image generating unit IGEU may receive demodulated data RDTAd via the demodulator DEMU in units of rows RD1 through RDn (see FIG. 5), or may receive the demodulated data RDTAd for all rows at once. When receiving the demodulated data RDTAd in units of rows, a storage unit (not shown) for temporarily storing a demodulating result for each row may be included in the image generating unit IGEU, and when receiving a demodulating result for all rows at once, the storage unit may be included in the demodulator DEMU.

The image generating unit IGEU may perform signal processing on the demodulated data RDTAd by converting the same from a wavelength domain to a depth domain. To this end, the image generating unit IGEU may perform background subtraction and k-linearization on the demodulated data RDTAd, and then perform a Fast Fourier transform (FFT), all of which are well known to one of ordinary skill in the art. However, signal processing of demodulated data RDTAd is not limited thereto, and the image generating unit IGEU may also perform signal processing to convert the demodulated data RDTAd from a wavelength domain to a depth domain using any of various other algorithms known to one of ordinary skill in the art. As a wavelength detected with respect to the subject 160 is processed as depth information, the image generating unit IGEU generates temporary tomographic images IMG1 through IMGn with respect to the subject 160.

The image generating unit IGEU may convert one piece of demodulated data RDTAd into at least two temporary tomographic images IMG1 through IMGn. As described above, of the at least two temporary tomographic images IMG1 through IMGn, a first temporary tomographic image IMG1 may be a reference temporary tomographic image.

FIG. 7 illustrates another example of an image processing unit IMGPU of FIG. 1.

Referring to FIGS. 1 and 7, unlike the image processing unit IMGPU of FIG. 6 that includes one demodulator DEMU, the image processing unit IMGPU includes at least two demodulators DEMU1 through DEMUm and an image generating unit IGEU. For example, the first demodulator DEMU1 may demodulate raw data RDTA into first demodulated data RDTAd1, and an m-th demodulator DEMUm (m is a natural number of 2 or greater) may demodulate raw data RDTA to generate m-th demodulated data RDTAdm. The at least two demodulators DEMU1 through DEMUm may each be the same as the demodulator DEMU of FIG. 6.

For example, each of at least two demodulators DEMU1 through DEMUm may be a VSB filter, which will be described later. In this case, the at least two demodulators DEMU1 through DEMUm that are VSB filters may be defined by different parameters.

The image generating unit IGEU performs signal processing on each of demodulated data RDTAd1 through RDTAdm to generate at least two temporary tomographic images IMG1 through IMGn. For example, if n and m are equal, the image generating unit IGEU may perform signal processing on first demodulated data RDTAd1 to generate a first temporary tomographic image IMG1, and may perform signal processing on m-th demodulated data RDTAdm to generate an n-th temporary tomographic image IMGn (m=n). The image generating unit IGEU of FIG. 7 may be the same as the image generating unit IGEU except for the number of pieces of received demodulated data.

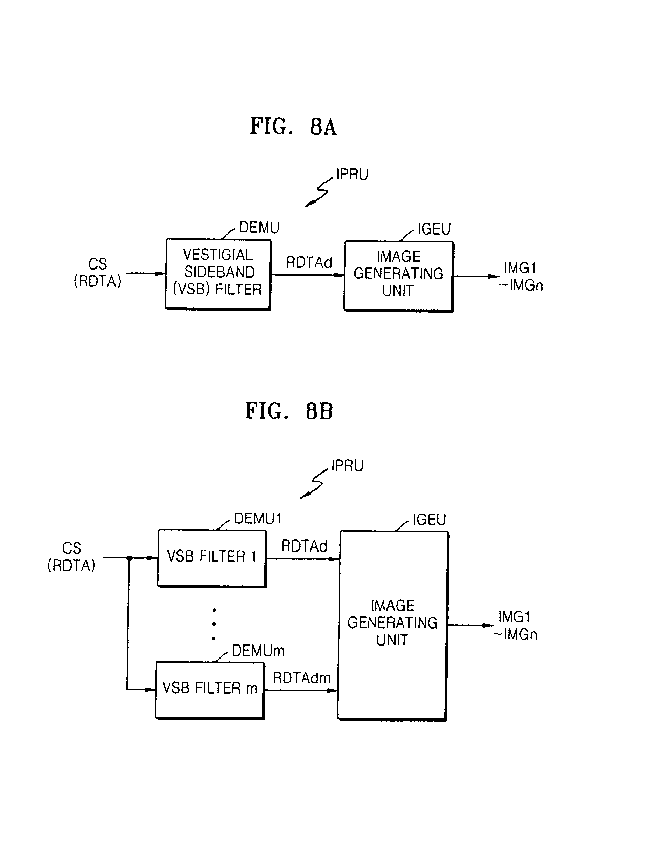

FIGS. 8A and 8B illustrate examples of the demodulators illustrated in FIGS. 6 and 7.

Referring to FIG. 8A, the demodulator DEMU of FIG. 6 may include a VSB filter. Likewise, referring to FIG. 8B, each of the demodulators DEMU1 through DEMUm of FIG. 7 may include a respective VSB filter 1 through m.

Hereinafter, a method of demodulating raw data using a demodulator including a VSB filter and performing a corresponding filtering operation will be described with regard to the demodulator DEMU of FIG. 6.

The demodulator DEMU in this example may perform a demodulating operation by adjusting parameters of a filter function defining a filtering operation of the demodulator DEMU using a filter having a fixed window size, and filtering raw data RDTA in units of rows. The demodulator DEMU may be set as a window having a fixed size corresponding to a window size signal XFW. For example, in response to the window size signal XFW, the demodulator DEMU may be set to have a size "a" ("a" is a constant), which is horizontally symmetrical with respect to a central value.

In this example, a fixed window size may be set with respect to the demodulator DEMU before demodulating raw data RDTA. For example, the demodulator DEMU may set a fixed window size in response to the fixed window size signal XFW that is input via the user interface unit 170 of FIG. 3 when the tomographic image generating apparatus 100 of FIG. 1 or FIG. 3 is set up or turned on. In this case, each time the tomographic image generating apparatus 100 of FIG. 1 or FIG. 3 is set up or turned on, the demodulator DEMU having different window sizes may be set. The window size may be set in units of taps.

When the demodulator DEMU is a VSB filter as in this example, a filtering operation of the demodulator DEMU may be expressed by Equation 2 below. In Equation 2, a y-function denotes a demodulation signal that is filtered and output, and an x-function denotes a signal representing each row of raw data RDTA. Also, in Equation 2, a sum of a $\delta$ function and an h function is a filter function. Also, N denotes a window size in Equation 2.

$$y(n) = (\delta(n) + h(n)) * x(n)$$
$$\approx x(n) + j\frac{2}{\pi}\sum_{k=0}^{N} coeffs(k) \times (x(n-2k-1) - x(n+2k+1)) \quad (2)$$

In Equation 2, it is assumed that the h function is expressed by Equation 3 below. When n is an even number, the value of the y-function is 0, and, thus the case with an even number is excluded in Equation 2.

$$h(n) = \begin{cases} 0 \ for \ even \ n \\ \dfrac{2}{n\pi} \ for \ odd \ n \end{cases} \quad (3)$$

Accordingly, in the demodulator DEMU of FIG. 6, multiplication by 1/2 of the window size N is performed. In addition, a coefficient coeffs of Equation 2 may be expressed by Equation 4 below. In Equation 4, T denotes a duration, and R denotes a roll-off value.

$$coeffs(k) = \frac{\sin\left(\dfrac{\pi k}{T}\right)}{\dfrac{\pi k}{T}} \cdot \frac{\cos\left(\dfrac{\pi R k}{T}\right)}{\left(1 - \dfrac{4R^2 k^2}{T^2}\right)} \cdot e^{\frac{2j\pi k}{4}} \quad (4)$$

As described above, a method of demodulating a coherence signal in this example may be performed by setting a waveform of the coherence signal with respect to a frequency domain by adjusting at least one parameter of a filter function that defines filtering.

For example, when a roll-off (R) value of a filter function is varied, an inclination of a rising section or a falling section in a waveform of raw data RDTA in a frequency domain may be varied. Accordingly, a flatness of a waveform of raw data RDTA of a frequency domain may be varied. Likewise, as a duration (T) of the filter function is varied, a flatness of the waveform may also vary.

Even when a fixed window size is provided so that the number of times of multiplication is kept constant, a flatness of a waveform of a demodulated coherence signal may be improved. Accordingly, in this example, to improve flatness, a window size, that is, the number of times of multiplication, does not need to be increased.

As described above, the at least two demodulators DEMU1 through DEMUm of FIG. 7 may be first through m VSB filters of FIG. 8B. In this case, the first through m VSB filters may be defined by different parameters. Accordingly, a plurality of pieces of demodulated data RDTAd through RDTAm that are demodulated from one piece of raw data RDTA of FIG. 8B using the first through m VSB filters may have different frequency response characteristics.

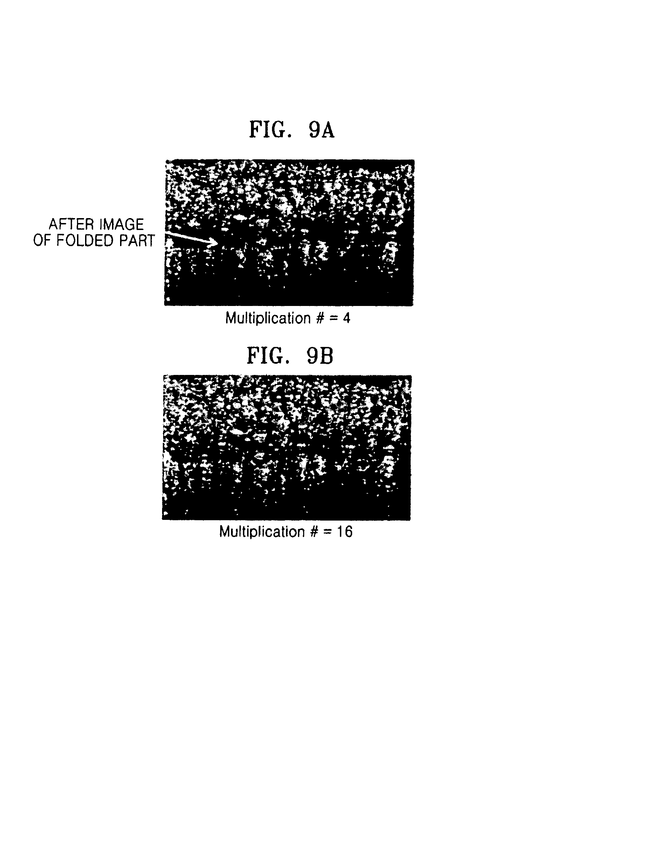

FIGS. 9A and 9B are images showing an example of a relationship between an increase in a complexity of filtering and a folding phenomenon. When flatness of a waveform of a demodulated signal is poor, a folding phenomenon as illustrated in FIG. 9A may occur. In this example, to prevent occurrence of the folding phenomenon of FIG. 9A without increasing the number of times of multiplication as illustrated in FIG. 9B, flatness of a waveform of a demodulated coherence signal may be improved by adjusting parameters of a filter function described above. Accordingly, in this example, consumption of time or resources for performing demodulating of a coherence signal may be minimized, and the folding phenomenon may also be effectively prevented.

In this example, when forming a tomographic image, a window size set for filtering is fixed in regard to demodulated data through filtering so that a quality of the tomographic image may be maintained and a complexity required for data demodulating may be reduced. In addition, in this example, when demodulating data, variation in a domain such as a time domain or a frequency domain does not occur, and thus the complexity may be further reduced.

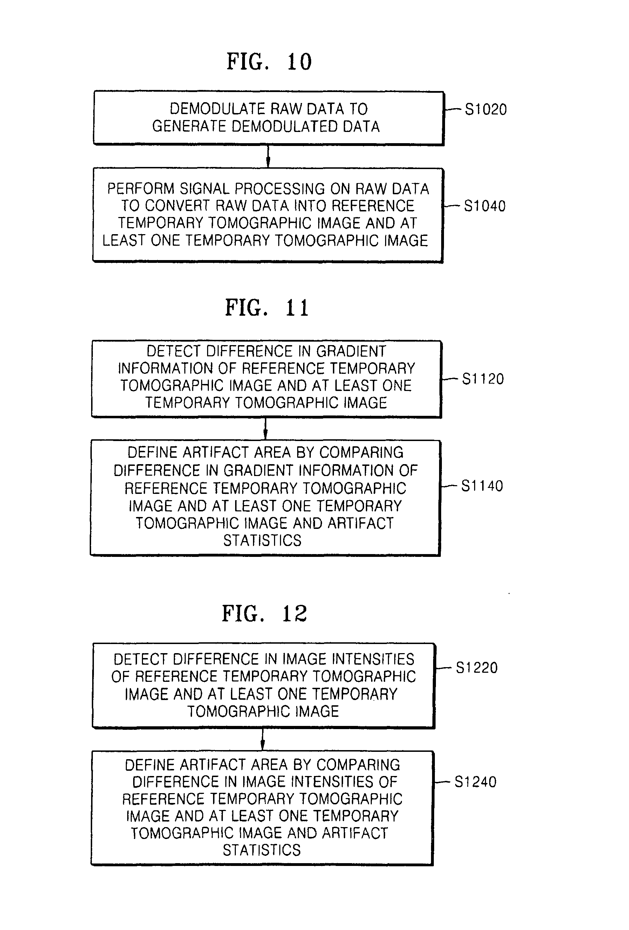

FIG. 10 is a flowchart illustrating an example of an operation of generating a reference temporary tomographic image and at least one temporary tomographic image illustrated in FIG. 2.

Referring to FIGS. 6, 7, and 10, the image processing unit IMGPU demodulates raw data RDTA to generate demodulated data RDTAd in operation S1020, and performs signal processing on the demodulated data RDTAd to convert the same into a reference temporary tomographic image and at least one temporary tomographic image. When the image processing unit IMGPU in this example generates at least two temporary tomographic images IMG1 through IMGn, as illustrated in FIG. 6, one piece of demodulated data RDTAd is generated using the demodulator DEMU, and the at least two temporary tomographic images IMG1 through IMGn are generated from the one piece of demodulated data RDTAd using the image generating unit IGEU, or as illustrated in FIG. 7, at least two pieces of demodulated data RDTAd1 through RDTAdm are generated using at least two demodulators DEMU1 through DEMUm, and the at least two temporary tomographic images IMG1 through IMGn are generated from the at least two pieces of demodulated data RDTAd1 through RDTAdm using the image generating unit IGEU.

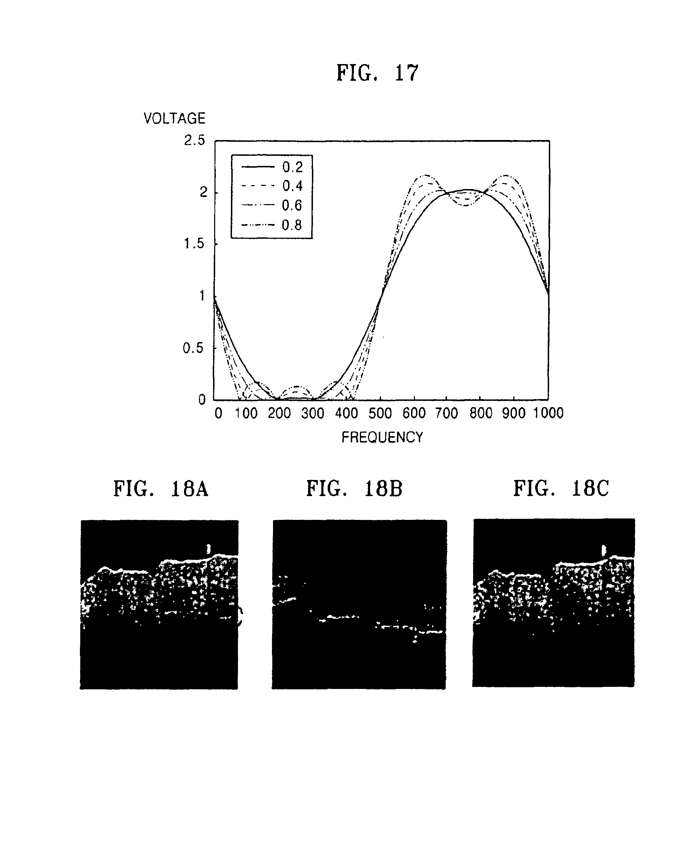

As described above, when at least two temporary tomographic images IMG1 through IMGn are generated, there may be a difference between frequency responses between the temporary tomographic images IMG1 through IMGn, and this may cause a artifact in an actual image in a saturation region according to a limited bandwidth of a signal as illustrated in FIG. 18A. By using the apparatus and method of generating a tomographic image of this example, the artifact may be efficiently detected and restored. This will be described in detail below.

FIG. 11 is a flowchart illustrating an example of an operation of detecting an artifact area illustrated in FIG. 2.

Referring to FIGS. 1, 2, and 11, as described above, in operation S260, an artifact area determining unit AADU detects an artifact area of a reference temporary tomographic image based on a result of comparing the reference temporary tomographic image and at least one temporary tomographic image and artifact statistics regarding whether an artifact area is present or not. For example, the artifact area determining unit AADU may detect an artifact area based on a difference in gradient information of the reference temporary tomographic image and at least one temporary tomographic image.

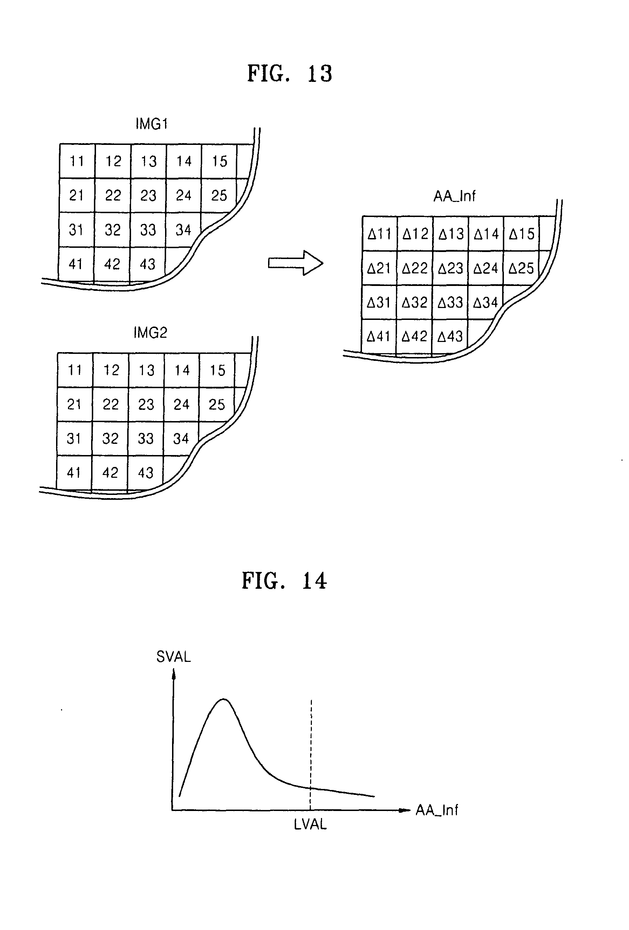

An edge area may be detected from the reference temporary tomographic image and at least one temporary tomographic image based on the gradient information. The gradient information may be calculated as expressed by Equation 5 below. In Equation 5, $\square$ denotes a differentiation operator, $\alpha$ denotes pixel values of temporary tomographic images IMG1 through IMGn, and x and y denote positions of corresponding pixels. As illustrated in FIG. 13 to be described later, each of the temporary tomographic images IMG1 through IMGn may be formed of a plurality of pixel values, i.e., may be a frame

$$\nabla^2 = \frac{\partial^2}{\partial x^2} + \frac{\partial^2}{\partial y^2}$$

$$\nabla^2 = \frac{4}{(\alpha+1)}\begin{bmatrix} \frac{\alpha}{4} & \frac{1-\alpha}{4} & \frac{\alpha}{4} \\ \frac{1-\alpha}{4} & -1 & \frac{1-a}{4} \\ \frac{\alpha}{4} & \frac{1-\alpha}{4} & \frac{\alpha}{4} \end{bmatrix} \qquad (5)$$

Referring further to FIGS. 1, 2, and 11, the artifact area determining unit AADU may define an artifact area based on a result of comparing a difference in gradient information of a reference temporary tomographic image and at least one temporary tomographic image and artifact statistics in operation S1140.

FIG. 12 is a flowchart illustrating another example of an operation of detecting an artifact area illustrated in FIG. 2.

Referring to FIGS. 1, 2, and 12, in regard to operation S260 in which the artifact area determining unit AADU detects an artifact area of a reference temporary tomographic image based on a result of comparing the reference temporary tomographic image and at least one temporary tomographic image and artifact statistics regarding_whether an artifact area exists, the artifact area determining unit AADU may detect an artifact area based on a difference in image intensities of a reference temporary tomographic image and at least one temporary tomographic image, unlike the method illustrated in FIG. 11. Image intensity may be calculated according to Equation 1 described above. In addition, by comparing a difference in image intensities of the reference temporary tomographic image and at least one temporary tomographic image and artifact statistics, an artifact area may be defined in operation S1160.

The method of detecting an artifact area using the method of FIG. 11 or FIG. 12 will now be described in detail.

FIG. 13 illustrates an example an operation of an artifact processing unit of FIG. 1 performing the method of FIG. 11 or FIG. 12.

Referring to FIG. 1, FIG. 11 or FIG. 12, and FIG. 13, the artifact area processing unit AFPU forms a reference temporary tomographic image IMG1 and a temporary tomographic image IMG2 that is not the reference temporary tomographic image IMG1 each having a plurality of pixels. Referring to FIG. 13, pixels are numbered in consideration of positions of rows and columns (x and y of Equation 5).

According to the method of FIG. 11, artifact area information AA_Inf is generated by detecting a difference between gradient information of pixels corresponding to the reference temporary tomographic image IMG1 and the temporary tomographic image IMG2 in operation S1120. For example, a difference Δ11 between gradient information of a pixel 11 of the reference temporary tomographic image IMG1 and the temporary tomographic image IMG2  may be detected as artifact area information AA_Inf. For example, when gradient values of pixels 11 of the reference temporary tomographic image IMG1 and the temporary tomographic images IMG2 are 30 and 35, the artifact area information AA_Inf may be set as 5, which is the difference Δ11 of the pixels 11. This also applies to other pixels. Accordingly, the artifact area information AA_Inf may include information about a difference between gradient values of pixels of the reference temporary tomographic image IMG1 and the temporary tomographic image IMG2.

According to the method of FIG. 12, in operation S1220, artifact area information AA_Inf is generated by detecting a difference between image intensities of corresponding pixels of the reference temporary tomographic image IMG1 and the temporary tomographic image IMG2. For example, a difference Δ11 between image intensities of the pixel 11 of the reference temporary tomographic image IMG1 and the pixel 11 of the temporary tomographic image IMG2 may be detected as artifact area information AA_Inf. For example, when image intensities of pixels 11 of the reference temporary tomographic image IMG1 and the temporary tomographic image IMG2 are 120 and 100, the artifact area information AA_Inf may be set as 20 with respect to the pixels 11, which is a difference Δ11. This also applies to other pixels. Accordingly, the artifact area information AA_Inf may include information about a difference between image intensities of pixels of the reference temporary tomographic image IMG1 and the temporary tomographic image IMG2.

FIG. 14 is a graph showing an example of a standard used by an artifact area determining unit of FIG. 1 to determine an artifact area.

Referring to FIGS. 1, 11 or 12, and 14, by comparing artifact area information AA_Inf detected according to operation S1120 or operation S1220 and artifact statistics SVAL of FIG. 14, an artifact area may be defined in operation S1140 or operation S1240. For example, pixels having an artifact area information AA_Inf greater than a threshold value LVAL are defined as an artifact area (pixels), and pixels having an artifact area information AA_Inf smaller than the threshold value LVAL are defined as not an artifact area (pixels). This is because a pixel for which a difference between a reference temporary tomographic image and at least one temporary tomographic image is great may have a distorted value. The artifact statistics SVAL and the threshold value LVAL may be obtained by sampling sample images.

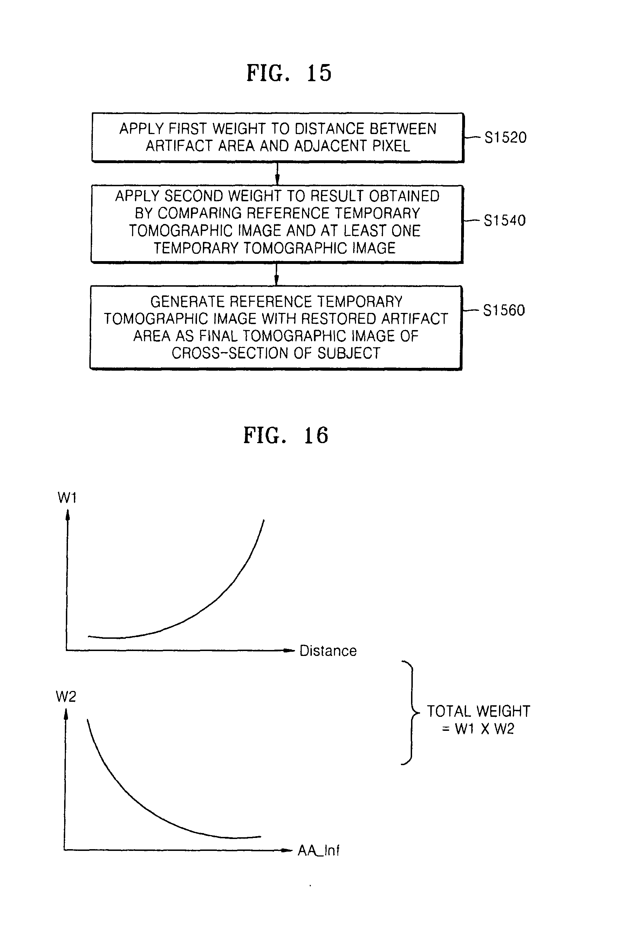

FIG. 15 is a flowchart illustrating an example of an operation of restoring an artifact area illustrated in FIG. 2, and FIG. 16 illustrates examples of graphs showing a first weight and a second weight of FIG. 15.

Referring to FIGS. 1, 2, 15, and 16, as described above, the artifact area restoring unit AASU in this example restores the artifact area in operation S280. For example, the artifact area restoring unit AASU applies a first weight W1 to a distance between an artifact area and a pixel adjacent to the artifact area in operation S1520, and applies a second weight W2 to artifact area information AA_Inf obtained by comparing a reference temporary tomographic image and at least one temporary tomographic image with respect to an area in operation S1540, and performs stitching on the artifact area included in the reference temporary tomographic image based on adjacent pixels, thereby restoring the artifact area.

The first weight W1 is proportional to a distance between an artifact area and an adjacent pixel. The closer the adjacent pixel is closer to the artifact area, the more the adjacent pixel may affect a pixel value of the artifact area. A second weight W2 is inversely proportional to artifact area information AA_Inf. As described above, when the artifact area information AA_Inf is greater than the threshold value LVAL of FIG. 14, the area corresponding to the artifact area information AA_Inf is defined as an artifact area, and thus, a small second weight W2 is applied to a pixel having large artifact area information AA_Inf so as to minimize the influence of an artifact.

FIG. 16 illustrates an example of restoring an artifact area by applying a total weight obtained by multiplying the first weight W1 by the second weight W2 to a pixel value of an artifact to restore the artifact area. The artifact area restoring unit AASU in this example may generate a reference temporary tomographic image including the restored artifact area as a final tomographic image of a cross-section of a subject in operation S1540.

By using the apparatus and method of generating a tomographic image of this example in which an artifact area is detected and restored according to the above-described method, an artifact remaining in a saturation area may be reduced, and thus a high-quality tomographic image may be generated.

Referring to FIG. 3 again, a final tomographic image TIMG that is generated with respect to a cross-section of the subject 160 and whose artifact is restored may be stored in a storage device STRU. Also, the final tomographic image TIMG may be displayed using a display unit DISU. The display unit DISU is a device that receives an image signal from the image processing unit IMGPU and outputs an image, and may be a separate device located outside the image processing unit IMGPU, or may be included in the image processing unit IMGPU. The image processing unit IMGPU may be manufactured as dedicated chips that perform functions of the elements as described above, or may be implemented as an dedicated program stored in a general-use central processing unit (CPU) or the storage device STRU.

FIG. 17 is a graph illustrating an example of adjusting a parameter of a filter function defining a VSB filter used in demodulating data.

Referring to FIGS. 1, 6, and 17, while maintaining a fixed window size, a roll-off value of a filter function defining a VSB filter of a demodulator DEMU used to restore raw data RDTA as demodulated data RDTAd may be varied to adjust a flatness of a waveform of the raw data RDTA as illustrated in FIG. 17. FIG. 17 illustrates an example in which the flatness of the waveform is optimum when the value of the roll-off R is 0.6. However, an optimized value of the roll-off R may change depending on an operational environment, and is not limited to the example of FIG. 17. In addition, the image generating unit IGEU may perform signal processing on demodulated data RDTAd to convert the same into at least two temporary tomographic images IMG1 through IMGn.

FIGS. 18A-18C illustrate an example of an operation of restoring an artifact.

FIG. 18A illustrates an example of a temporary tomographic image of the at least two temporary tomographic images IMG1 through IMGn. The temporary tomographic images IMG1 through IMGn may include an artifact as illustrated in FIG. 18A. The artifact area determining unit AADU of a frequency domain may define an artifact area (the area enclosed by the dashed-line oval) of FIG. 18A in which an artifact area is included to thereby generate artifact area information AA_Inf. The artifact area restoring unit AASU performs a restoration operation by applying a weight according to the above-described method as illustrated in FIG. 18B. As a result, as illustrated in FIG. 18C, the artifact of FIG. 18A may be restored.

According to the examples described above, tomographic images may be generated accurately without increasing a complexity of a calculation required for generation of the tomographic images.

The image processing unit IMGPU, the artifact area determining unit AADU, the artifact processing unit AFPU, the artifact area restoring unit AASU, the demodulator DEMU and the image generating unit IGEU of FIG. 6, the plurality of demodulators DEMU1 through DEMUm the image generating unit IGEU of FIG. 7, the vestigial sideband (VSB) filter VSB and the image generating unit IGEU of FIG. 8A, the plurality of vestigial sideband (VSB) filters and the image generating unit IGEU of FIG. 8B described above that perform the operations illustrated in FIGS. 2, 10-12, and 15 may be implemented using one or more hardware components, one or more software components, or a combination of one or more hardware components and one or more software components.

A hardware component may be, for example, a physical device that physically performs one or more operations, but is not limited thereto. Examples of hardware components include resistors, capacitors, inductors, power supplies, frequency generators, operational amplifiers, power amplifiers, low-pass filters, high-pass filters, band-pass filters, analog-to-digital converters, digital-to-analog converters, and processing devices.

A software component may be implemented, for example, by a processing device controlled by software or instructions to perform one or more operations, but is not limited thereto. A computer, controller, or other control device may cause the processing device to run the software or execute the instructions. One software component may be implemented by one processing device, or two or more software components may be implemented by one processing device, or one software component may be implemented by two or more processing devices, or two or more software components may be implemented by two or more processing devices.

A processing device may be implemented using one or more general-purpose or special-purpose computers, such as, for example, a processor, a controller and an arithmetic logic unit, a digital signal processor, a microcomputer, a field-programmable array, a programmable logic unit, a microprocessor, or any other device capable of running software or executing instructions. The processing device may run an operating system (OS), and may run one or more software applications that operate under the OS. The processing device may access, store, manipulate, process, and create data when running the software or executing the instructions. For simplicity, the singular term "processing device" may be used in the description, but one of ordinary skill in the art will appreciate that a processing device may include multiple processing elements and multiple types of processing elements. For example, a processing device may include one or more processors, or one or more processors and one or more controllers. In addition, different processing configurations are possible, such as parallel processors or multi-core processors.

A processing device configured to implement a software component to perform an operation A may include a processor programmed to run software or execute instructions to control the processor to perform operation A. In addition, a processing device configured to implement a software component to perform an operation A, an operation B, and an operation C may have various configurations, such as, for example, a processor configured to implement a software component to perform operations A, B, and C; a first processor configured to implement a software component to perform operation A, and a second processor configured to implement a software component to perform operations B and C; a first processor configured to implement a software component to perform operations A and B, and a second processor configured to implement a software component to perform operation C; a first processor configured to implement a software component to perform operation A, a second processor configured to implement a software component to perform operation B, and a third processor configured to implement a software component to perform operation C; a first processor configured to implement a software component to perform operations A, B, and C, and a second processor configured to implement a software component to perform operations A, B, and C, or any other configuration of one or more processors each implementing one or more of operations A, B, and C. Although these examples refer to three operations A, B, C, the number of operations that may implemented is not limited to three, but may be any number of operations required to achieve a desired result or perform a desired task.

Software or instructions for controlling a processing device to implement a software component may include a computer program, a piece of code, an instruction, or some combination thereof, for independently or collectively instructing or configuring the processing device to perform one or more desired operations. The software or instructions may include machine code that may be directly executed by the processing device, such as machine code produced by a compiler, and/or higher-level code that may be executed by the processing device using an interpreter. The software or instructions and any associated data, data files, and data structures may be embodied permanently or temporarily in any type of machine, component, physical or virtual equipment, computer storage medium or device, or a propagated signal wave capable of providing instructions or data to or being interpreted by the processing device. The software or instructions and any associated data, data files, and data structures also may be distributed over network-coupled computer systems so that the software or instructions and any associated data, data files, and data structures are stored and executed in a distributed fashion.

For example, the software or instructions and any associated data, data files, and data structures may be recorded, stored, or fixed in one or more non-transitory computer-readable storage media. A non-transitory computer-readable storage medium may be any data storage device that is capable of storing the software or instructions and any associated data, data files, and data structures so that they can be read by a computer system or processing device. Examples of a non-transitory computer-readable storage medium include read-only memory (ROM), random-access memory (RAM), flash memory, CD-ROMs, CD-Rs, CD+Rs, CD-RWs, CD+RWs, DVD-ROMs, DVD-Rs, DVD+Rs, DVD-RWs, DVD+RWs, DVD-RAMs, BD-ROMs, BD-Rs, BD-R LTHs, BD-REs, magnetic tapes, floppy disks, magneto-optical data storage devices, optical data storage devices, hard disks, solid-state disks, or any other non-transitory computer-readable storage medium known to one of ordinary skill in the art.

Functional programs, codes, and code segments for implementing the examples disclosed herein can be easily constructed by a programmer skilled in the art to which the examples pertain based on the drawings and their corresponding descriptions as provided herein.

While this disclosure includes specific examples, it will be apparent to one of ordinary skill in the art that various changes in form and details may be made in these examples without departing from the scope of the claims.

For example, as illustrated in FIG. 7, when generating a plurality of pieces of demodulated data RDTAd1 through RDTAdm, a plurality of demodulators DEMU1 through DEMUm are used, but other methods of generating a plurality of demodulated data may be used. For example, a plurality of demodulated data may be sequentially generated using a single demodulator.

The examples described herein are to be considered in descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents. Therefore, the scope of the invention is defined not by the detailed description, but by the claims.

**Claims**

1. A method of generating a tomographic image, the method comprising:

   detecting a coherence signal that is phase-modulated in a first direction with respect to a cross-section of a subject and includes cross-sectional information of the subject as raw data about the subject;
   generating a reference temporary tomographic image and at least one temporary tomographic image by performing signal processing on the raw data;
   detecting an artifact area of the reference temporary tomographic image based on a result of comparing the reference temporary tomographic image with the at least one temporary tomographic image and based on artifact statistics regarding whether an artifact exists; and
   restoring the artifact area in response to a result of the detecting.

2. The method of claim 1, wherein the generating of a reference temporary tomographic image and at least one temporary tomographic image by performing signal processing on the raw data comprises:

   generating demodulated data by demodulating the raw data; and
   performing signal processing on the demodulated data to convert the demodulated data into the reference temporary tomographic image and the at least one temporary tomographic image.

3. The method of claim 2, wherein the generating of demodulated data comprises adjusting at least one parameter of a filter function defining a vestigial sideband (VSB) filter;
   wherein the at least one parameter preferably is a duration or a roll-off value of the filter function.

4. The method of claim 2 or 3, wherein the generating of demodulated data comprises generating the raw data into at least two pieces of demodulated data;
   wherein the performing of signal processing on the demodulated data preferably comprises performing signal processing on the at least two pieces of demodulated data to convert the at least two pieces of demodulated data into the reference temporary tomographic image and the at least one temporary tomographic image.

5. The method of claim 4, wherein the generating of demodulated data comprises demodulating the at least two pieces of demodulated data using different VSB filters defined by different parameters.

6. The method of claim 2 or 3, wherein the performing of signal processing on the demodulated data comprises performing signal processing on the demodulated data to convert the one demodulated data into the reference temporary tomographic image and the at least one temporary tomographic image.

7. The method of claim 2 or any one of claims 3 to 6 in combination with claim 2,
   wherein the performing of the signal processing on the demodulated data comprises performing signal processing on the demodulated data in a second direction that is perpendicular to the first direction.

8. The method of claim 2 or any one of claims 3 to 6 in combination with claim 2,
   wherein the demodulated data is in a wavelength domain; and
   the performing of signal processing on the demodulated data comprises converting the demodulated data in the wavelength domain into depth information about the subject.

9. The method of any one of claims 1 to 8, wherein the detecting of an artifact area of the reference temporary tomographic image comprises defining the artifact area based on a difference between gradient information of the reference temporary tomographic image and gradient information of the at least one temporary tomographic image; or wherein the detecting of an artifact area of the reference temporary tomographic image comprises defining the artifact area based on a difference between image intensities of the reference temporary tomographic image and image intensities of the at least one temporary tomographic image.

10. The method of any one of claims 1 to 9, wherein the restoring of the artifact area comprises restoring the artifact area based on a first weight that is applied to a distance between the artifact area and at least one adjacent pixel and a second weight that is applied to a result of comparing the reference temporary tomographic image with the at least one temporary tomographic image with respect to the artifact area.

11. The method of claim 1, further comprising generating the reference temporary tomographic image with the restored artifact as a final tomographic image with respect to the cross-section of the subject; and/or wherein the method is an optical coherent tomographic (OCT) method.

12. A non-transitory computer readable storage medium storing a program for controlling a computer to perform the method of generating a tomographic image of any one of claims 1 to 11.

13. An apparatus for generating a tomographic image, the apparatus comprising:

   an image processing unit configured to receive raw data corresponding to a coherence signal including sectional information of the subject that is phase-modulated in a first direction with respect to a cross-section of the subject, and generate a reference temporary tomographic image and at least one temporary tomographic image by performing signal processing on the raw data; and
   an artifact processing unit configured to detect an artifact area of the reference temporary tomographic based on a result of comparing the reference temporary tomographic image with the at least one temporary tomographic image and based on artifact statistics regarding whether an artifact exists, and restore the artifact area.

14. The apparatus of claim 13, wherein the artifact processing unit comprises:

   an artifact area determining unit configured to define the artifact area based on a difference between gradient information of the reference temporary tomographic image and gradient information of the at least one temporary tomographic image; and
   an artifact area restoring unit configured to restore the artifact area based on a first weight that is applied to a distance between the artifact area and at least one adjacent pixel and a second weight that is applied to a result of comparing the reference temporary tomographic image with the at least one temporary tomographic image with respect to the artifact area.

15. The apparatus of claim 13 or 14, wherein the artifact area determining unit comprises at least one vestigial sideband (VSB) filter; and/or wherein the artifact area restoring unit is further configured to generate the reference temporary tomographic with the restored artifact area as a final tomographic image with respect to the cross-section of the subject.

# FIG. 1

# FIG. 2

DETECT COHERENCE SIGNAL INCLUDING CROSS-SECTION INFORMATION OF SUBJECT THAT IS PHASE-MODULATED IN FIRST DIRECTION WITH RESPECT TO CROSS-SECTION OF SUBJECT, AS RAW DATA ABOT SUBJECT — S220

PERFORM SIGNAL PROCESSING ON RAW DATA TO GENERATE REFERENCE TEMPORARY TOMOGRAPHIC IMAGE AND AT LEAST ONE TEMPORARY TOMOGRAPHIC IMAGE — S240

DETECT ARTIFACT AREA OF REFERENCE TEMPORARY TOMOGRAPHIC IMAGE BASED ON RESULT OF COMPARING REFERENCE TEMPORARY TOMOGRAPHIC IMAGE AND AT LEAST ONE TEMPORARY TOMOGRAPHIC IMAGE AND STATISTICS WITH RESPECT TO ARTIFACT AREA — S260

RESTORE ARTIFACT AREA — S280

# FIG. 3

# FIG. 4

# FIG. 5

# FIG. 6

IPRU

CS
(RDTA) → | DEMU<br>DEMODULATING UNIT | → RDTAd → | IGEU<br>IMAGE GENERATING UNIT | → IMG1<br>~IMGn

# FIG. 7

IPRU

CS
(RDTA) → | DEMU1<br>DEMODULATING UNIT | → RDTAd

| DEMUm<br>DEMODULATING UNIT | → RDTAdm

| IGEU<br>IMAGE GENERATING UNIT | → IMG1<br>~IMGn

# FIG. 8A

IPRU

CS
(RDTA) → VESTIGIAL SIDEBAND (VSB) FILTER [DEMU] — RDTAd → IMAGE GENERATING UNIT [IGEU] → IMG1 ~IMGn

# FIG. 8B

IPRU

CS
(RDTA) → VSB FILTER 1 [DEMU1] — RDTAd → IMAGE GENERATING UNIT [IGEU] → IMG1 ~IMGn

⋮

→ VSB FILTER m [DEMUm] — RDTAdm →

## FIG. 9A

AFTER IMAGE
OF FOLDED PART

Multiplication # = 4

## FIG. 9B

Multiplication # = 16

## FIG. 10

DEMODULATE RAW DATA TO
GENERATE DEMODULATED DATA — S1020

PERFORM SIGNAL PROCESSING ON RAW DATA
TO CONVERT RAW DATA INTO REFERENCE
TEMPORARY TOMOGRAPHIC IMAGE AND AT — S1040
LEAST ONE TEMPORARY TOMOGRAPHIC IMAGE

## FIG. 11

DETECT DIFFERENCE IN GRADIENT
INFORMATION OF REFERENCE TEMPORARY
TOMOGRAPHIC IMAGE AND AT LEAST ONE — S1120
TEMPORARY TOMOGRAPHIC IMAGE

DEFINE ARTIFACT AREA BY COMPARING
DIFFERENCE IN GRADIENT INFORMATION OF
REFERENCE TEMPORARY TOMOGRAPHIC
IMAGE AND AT LEAST ONE TEMPORARY — S1140
TOMOGRAPHIC IMAGE AND ARTIFACT
STATISTICS

## FIG. 12

DETECT DIFFERENCE IN IMAGE INTENSITIES
OF REFERENCE TEMPORARY TOMOGRAPHIC
IMAGE AND AT LEAST ONE TEMPORARY — S1220
TOMOGRAPHIC IMAGE

DEFINE ARTIFACT AREA BY COMPARING
DIFFERENCE IN IMAGE INTENSITIES OF
REFERENCE TEMPORARY TOMOGRAPHIC
IMAGE AND AT LEAST ONE TEMPORARY — S1240
TOMOGRAPHIC IMAGE AND ARTIFACT
STATISTICS

# FIG. 13

IMG1

| 11 | 12 | 13 | 14 | 15 |
|----|----|----|----|----|
| 21 | 22 | 23 | 24 | 25 |
| 31 | 32 | 33 | 34 |  |
| 41 | 42 | 43 |  |  |

AA_Inf

| Δ11 | Δ12 | Δ13 | Δ14 | Δ15 |
|----|----|----|----|----|
| Δ21 | Δ22 | Δ23 | Δ24 | Δ25 |
| Δ31 | Δ32 | Δ33 | Δ34 |  |
| Δ41 | Δ42 | Δ43 |  |  |

IMG2

| 11 | 12 | 13 | 14 | 15 |
|----|----|----|----|----|
| 21 | 22 | 23 | 24 | 25 |
| 31 | 32 | 33 | 34 |  |
| 41 | 42 | 43 |  |  |

# FIG. 14

# FIG. 15

APPLY FIRST WEIGHT TO DISTANCE BETWEEN ARTIFACT AREA AND ADJACENT PIXEL ——— S1520

APPLY SECOND WEIGHT TO RESULT OBTAINED BY COMPARING REFERENCE TEMPORARY TOMOGRAPHIC IMAGE AND AT LEAST ONE TEMPORARY TOMOGRAPHIC IMAGE ——— S1540

GENERATE REFERENCE TEMPORARY TOMOGRAPHIC IMAGE WITH RESTORED ARTIFACT AREA AS FINAL TOMOGRAPHIC IMAGE OF CROSS-SECTION OF SUBJECT ——— S1560

# FIG. 16

W1

Distance

W2

AA_Inf

TOTAL WEIGHT
= W1 X W2

## FIG. 17

## FIG. 18A

## FIG. 18B

## FIG. 18C

Europäisches
Patentamt

European
Patent Office

Office européen
des brevets

# EUROPEAN SEARCH REPORT

**Application Number**

EP 13 17 0094

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/239531 A1 (KLEEN MARTIN [DE] ET AL) 26 October 2006 (2006-10-26) * the whole document * ----- | 1,12,13 | INV.<br>A61B5/00<br>G01B9/02 |
| A | WO 2009/149131 A1 (LIGHTLAB IMAGING INC [US]; SCHMITT JOSEPH M [US]; XU CHENYANG [US]) 10 December 2009 (2009-12-10) * paragraphs [0012], [0045] - [0048], [0068] * ----- | 1-15 | |
| A | WO 2006/127952 A2 (SOUTHWEST SCIENCES INC [US]; VAKHTIN ANDREI B [US]; KANE DANIEL J [US]) 30 November 2006 (2006-11-30) * paragraphs [0019], [0035] - [0047] * ----- | 1-15 | |

| | |
|---|---|
| | **TECHNICAL FIELDS SEARCHED (IPC)**<br><br>A61B<br>G01B |

The present search report has been drawn up for all claims

| Place of search | Date of completion of the search | Examiner |
|---|---|---|
| Berlin | 6 September 2013 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 13 17 0094

This annex lists the patent family members relating to the patent documents cited in the above-mentioned European search report.
The members are as contained in the European Patent Office EDP file on
The European Patent Office is in no way liable for these particulars which are merely given for the purpose of information.

06-09-2013

| Patent document cited in search report | Publication date | Patent family member(s) | Publication date |
|---|---|---|---|
| US 2006239531 A1 | 26-10-2006 | DE 102005019369 B3<br>JP 5121163 B2<br>JP 2006314781 A<br>US 2006239531 A1 | 16-11-2006<br>16-01-2013<br>24-11-2006<br>26-10-2006 |
| WO 2009149131 A1 | 10-12-2009 | CN 102046071 A<br>EP 2293714 A1<br>JP 2011521747 A<br>US 2009306520 A1<br>WO 2009149131 A1 | 04-05-2011<br>16-03-2011<br>28-07-2011<br>10-12-2009<br>10-12-2009 |
| WO 2006127952 A2 | 30-11-2006 | EP 1888999 A2<br>US 2006290939 A1<br>WO 2006127952 A2 | 20-02-2008<br>28-12-2006<br>30-11-2006 |

EPO FORM P0459

For more details about this annex : see Official Journal of the European Patent Office, No. 12/82